# EUROPEAN PATENT APPLICATION

(11) **EP 3 859 014 A1**
(43) Date of publication of application: **04.08.2021**
(21) Application number: 18934817.0
(22) Date of filing: 27.09.2018
(51) Int. Cl.: C12Q 1/6869

(54) **METHOD FOR CONSTRUCTING SEQUENCING LIBRARY, OBTAINED SEQUENCING LIBRARY AND SEQUENCING METHOD**

(71) Applicant: BGI Shenzhen, Shenzhen, Guangdong 518083 (CN)
(72) Inventor: CUI, Luman, Shenzhen, Guangdong 518083 (CN); FAN, Fei, Shenzhen, Guangdong 518083 (CN); ZHANG, Wenwei, Shenzhen, Guangdong 518083 (CN); LONG, Zhou, Shenzhen, Guangdong 518083 (CN); WANG, Weimao, Shenzhen, Guangdong 518083 (CN); WANG, Ou, Shenzhen, Guangdong 518083 (CN)
(74) Representative: Habermann, Hruschka & Schnabel
(86) International application number: PCT/CN2018/107980
(87) International publication number: WO 2020/061903

(57) **Abstract**

A method for constructing a sequencing library, and an obtained sequencing library and a sequencing method. The construction method includes: cyclizing a linear nucleic acid molecule to form a circular nucleic acid molecule, performing rolling circle amplifying to obtain a multi-copy a long-fragment nucleic acid molecule, and then synthesizing a complementary strand to obtain a double-stranded long-fragment nucleic acid molecule; mixing and incubating with a transposition complex to form a long fragment nucleic acid molecule carrying the transposition complex, and mixing and incubating with a solid-phase carrier having a molecular barcode sequence to link the molecular barcode sequence to a transposon sequence on the transposition complex; releasing a transposase of the transposition complex from the long-fragment nucleic acid molecule, and simultaneously breaking the long fragment nucleic acid molecule into short-fragment nucleic acid molecules connected with the transposon sequence and molecular barcode sequence; performing polymerase chain amplification on the short nucleic acid molecule to obtain a sequencing library.

## Description

### TECHNICAL FIELD

The present invention relates to the technical field of sequencing, and particularly, to a method for constructing a sequencing library, a sequencing library obtained thereby, and a sequencing method.

### BACKGROUND

Gene sequencing technology has become one of the important methods in modern biological research with the rapid development of molecular biology. It is widely used in reproductive health, genetic risk assessment, tumor prevention, screening, diagnosis, treatment and prognosis. Gene sequencing technology can truly reflect all the genetic information of DNAs in the genome, and reveal the mechanism and development process of tumor more comprehensively. Therefore, it plays a very important role in the scientific research of tumor. The first-generation sequencing technology is the dideoxy nucleotide terminal termination method invented by Sanger et al. in 1977 and the chemical degradation method invented by Gilbert et al.; the second-generation sequencing technology includes 454 technology by Roche, Solexa technology by Illumina, and SOLiD technology by ABI and DNA nanoball (DNB) sequencing technology by BGI, etc.; and the third-generation sequencing technology refer to the single molecule sequencing technology by Helicos and Pacbio. Since the third-generation sequencing technology has higher requirements for libraries and needs higher sequencing costs, the second-generation sequencing technology is currently the most widely used. For example, the whole genome sequencing technology is applied to non-invasive prenatal gene detection, target region capture sequencing technology is used to detect tumor targeted drug genes, single cell genome and transcriptome sequencing technology is used to study the heterogeneity and mechanism of occurrence and development of tumor tissue, and long fragment sequencing technology should be applied to non-invasive thalassemia detection research. Various clinical tests and basic research are carried out on the second-generation sequencing platform. The emergence of high-throughput sequencing technology has brought revolutionary changes to molecular detection in clinical laboratories. However, for efficient testing and wide clinical application, it is an important issue to build a higher quality library and obtain better sequencing data.

At present, most genome sequencing libraries are constructed by randomly breaking long double-stranded DNA fragments into small fragments of hundreds bp by means of physical manners or enzyme digestion, then repairing the terminals, adding "A" and "linker", amplifying with PCR and the like, so as to finally obtain a library for sequencing. Transcriptome sequencing technology uses oligo dT (polythymidine deoxynucleotide) or random primers to capture mRNA for reverse transcription and double-strand synthesis to obtain double-stranded cDNA molecules, and the subsequent library construction scheme is basically the same as that of genomic library construction plan. However, the fragmentation process of this library construction method cannot obtain long-fragment gene information, and it will lose some information, which increases the difficulty of genome assembly for de novo sequencing of new species. In addition, when the target region is amplified in the region to be detected, the primer- or probe-binding sites may be reduced due to the breakage of the region to be detected, thereby reducing the capture efficiency. In addition, the PCR amplification in the process of library construction is exponential, and the DNA distribution is biased due to the fragmentation, which will be amplified by PCR amplification, thereby leading to uneven coverage of sequencing data.

In addition, there is also a library construction method of short-read sequence co-barcoding using virtual compartment labeling, in which all short-read sequences of a long fragment of DNA are labelled with the same barcode, and the original information of the long fragment is obtained according to the barcode splicing. This method can be used to detect ultra-long DNA fragments. However, for fragments below 10kb, such as conventionally extracted genomic DNA, highly degraded Formalin-Fixed and Paraffin-Embedded (FFPE) samples and full-length mRNA fragments, due to the short length of single copy and the restriction of transposase in the process of library construction, this method has a small coverage and is not conducive to genome assembly and de novo sequencing of new species, thereby limiting the application scope of this technology.

### SUMMARY

The present disclosure provides a method for constructing a sequencing library, which overcomes the problems of single-copy short fragments and limitation of the role of transposase in the library construction process.

According to a first aspect, the present disclosure provides a method for constructing a sequencing library, the method including:
cyclizing a linear nucleic acid molecule to form a circular nucleic acid molecule, performing a rolling circle amplification using the circular nucleic acid molecule as a template to obtain a multi-copy long-fragment nucleic acid molecule, and then synthesizing a complementary strand to obtain a double-stranded long-fragment nucleic acid molecule;
mixing and incubating the long-fragment nucleic acid molecule with a transposition complex to form a long-fragment nucleic acid molecule with the transposition complex, wherein the transposition complex includes a transposon sequence and a transposase; and then mixing and incubating with substrate carried a molecular barcode sequence so as to connect the molecular barcode sequence to the transposon sequence of the transposition complex;
releasing the transposase of the transposition complex from the long-fragment nucleic acid molecule, to break the long-fragment nucleic acid molecule into a plurality of short-fragment nucleic acid molecules, wherein each of the plurality of short-fragment nucleic acid molecules is connected with the transposon sequence and the molecular barcode sequence, and the plurality of short-fragment nucleic acid molecules derived from the same long-fragment nucleic acid molecule is connected with the same molecular barcode sequence.

According to the first aspect, the present disclosure further provides a method for constructing a sequencing library, the method including:
cyclizing a linear nucleic acid molecule to form a circular nucleic acid molecule, performing a rolling circle amplification using the circular nucleic acid molecule as a template to obtain a multi-copy long-fragment nucleic acid molecule, and then synthesizing a complementary strand to obtain a double-stranded long-fragment nucleic acid molecule;
connecting a molecular barcode sequence on a solid-phase carrier having the molecular barcode sequence to a transposon sequence, then mixing and incubating with a transposase and optionally another transposon sequence in such a manner that the transposon sequence and the transposase form a transposition complex to obtain the solid-phase carrier having the molecular barcode sequence and the transposition complex, and then mixing and incubating with the long-fragment nucleic acid molecule to connect the transposition complex with the long-fragment nucleic acid molecule;
releasing the transposase of the transposition complex from the long-fragment nucleic acid molecule, to break the long-fragment nucleic acid molecule into a plurality of short-fragment nucleic acid molecules, wherein each of the plurality of short-fragment nucleic acid molecules is connected with the transposon sequence and the molecular barcode sequence, and the plurality of short-fragment nucleic acid molecules derived from the same long-fragment nucleic acid molecule is connected with the same molecular barcode sequence.

As a preferable technical solution, the above method further includes: amplifying, through polymerase chain reaction, the short-fragment nucleic acid molecule connected with the transposon sequence and the molecular barcode sequence in such a manner that each molecule of an amplification product includes the short-fragment nucleic acid molecule, the transposon sequence and the molecular barcode sequence.

As a preferable technical solution, the linear nucleic acid molecule is a nucleic acid molecule in a Formalin-Fixed and Paraffin-Embedded sample, or a cDNA sequence after reverse transcription of a full-length mRNA, or a full-length DNA sequence after reverse transcription of 18S rRNA and 16S rRNA, or a genomic DNA fragment sequence, or a full-length sequence of a mitochondrial or small genome sequence, or an amplicon sequence of a target region of a genomic DNA.

As a preferable technical solution, the linear nucleic acid molecule is cyclized to form the circular nucleic acid molecule by connecting a linker sequence at two terminals and forming complementary sticky terminals at the two terminals, and then the multi-copy long-fragment nucleic acid molecule is obtained through the rolling circle amplification using the circular nucleic acid molecule as the template and a sequence complementary to the linker sequence as a primer.

As a preferable technical solution, the linker sequence includes a U base site, and the complementary sticky terminals are formed by USER enzyme digestion; or the linker sequence includes an enzyme digestion site, and the complementary sticky terminals are formed by enzyme digestion.

As a preferable technical solution, the transposition complex includes a pair of transposon sequences that are identical to or different from each other.

As a preferable technical solution, the transposition complex includes the pair of transposon sequences that are different from each other, each transposon sequence includes a sense strand and an antisense strand, wherein in one transposon sequence of the pair of transposon sequences, the sense strand is connectable with the molecular barcode sequence, and the antisense strand has a U base site, which is removable by USER enzyme digestion to facilitate a subsequent polymerase chain reaction amplification.

As a preferable technical solution, the transposition complex includes the pair of transposon sequences that are identical to each other, each transposon sequence includes a sense strand and an antisense strand, and the sense strand of each transposon sequence is connectable with the molecular barcode sequence, and the antisense strand has a U base site, which can be removed by USER enzyme digestion.

As a preferable technical solution, after the transposase of the transposition complex is released from the long-fragment nucleic acid molecule to break the long-fragment nucleic acid molecule into the plurality of short-fragment nucleic acid molecules, a second linker sequence is connected at a gap where the transposon sequence is connected to the short-fragment nucleic acid molecules, and then polymerase chain reaction amplification is performed.

As a preferable technical solution, the solid phase carrier having the molecular barcode sequence includes more than two molecular barcode sequences.

As a preferable technical solution, the molecular barcode sequence is added to the solid phase carrier by connecting with the linker sequence on the solid phase carrier.

As a preferable technical solution, the solid phase carrier includes more than two molecular barcode sequences, and the more than two molecular barcode sequences are sequentially connected and added to the solid phase carrier to form a combined molecular barcode including the more than two molecular barcode sequences.

As a preferable technical solution, before mixing and incubating the long-fragment nucleic acid molecule having the transposition complex with the solid phase carrier having the molecular barcode sequence, a transposition complex-capturing sequence is added to the solid phase carrier having the molecular barcode sequence to complementarily connect to the molecular barcode sequence; the transposition complex-capturing sequence is then mixed and incubated with the long-fragment nucleic acid molecule having the transposition complex in such a manner that the transposition complex-capturing sequence is complementary to the molecular barcode sequence and the transposon sequence on the transposition complex to form a bridge therebetween, and the molecular barcode sequence is connected with the transposon sequence on the transposition complex by a ligase.

As a preferable technical solution, when the long-fragment nucleic acid molecule having the transposition complex is mixed and incubated with the solid phase carrier having the molecular barcode sequence, each solid phase carrier forms a virtual division in such a manner that one solid phase carrier captures one long-fragment nucleic acid molecule having the transposition complex and connects the molecular barcode sequence with the transposon sequence of the transposition complex.

As a preferable technical solution, when the solid-phase carrier having the molecular barcode sequence and the transposition complex is mixed and incubated with the long-fragment nucleic acid molecule, each solid-phase carrier forms a virtual division in such a manner that one solid-phase carrier captures one long-fragment nucleic acid molecule.

According to a second aspect, the present disclosure provides a sequencing library prepared by the method according to the first aspect.

According to a third aspect, the present disclosure provides a sequencing method, including sequencing the sequencing library prepared according to the first aspect. In addition to using the sequencing library prepared by the present disclosure, other aspects of the sequencing method of the present disclosure can be carried out according to the common sequencing methods in the art, including the second-generation sequencing technology, such as 454 technology by Roche, Solexa technology by Illumina, SOLiD technology by ABI, and DNB sequencing technology by BGI, etc.; as well as the third-generation sequencing technology, such as the single molecule sequencing technology of Helicos company and Pacbio.

As a preferable technical solution, said sequencing is selected from full-length transcript assembly sequencing, full-length sequencing of 18S rRNA or 16S rRNA, full-length sequencing of mitochondria, or long-fragment amplicon sequencing.

In the method of the present disclosure, a linear nucleic acid molecule is cyclized to form a circular nucleic acid molecule, then a multi-copy long-fragment nucleic acid molecule is obtained by rolling circle amplification, a complementary strand is further synthesized to obtain a double-stranded long-fragment nucleic acid molecule, then virtual compartment and rapid enzyme reaction are utilized to label the nucleic acid molecules in the same virtual compartment with the same molecular barcode, and then conventional library construction and sequencing are carried out. After sequencing, based on the molecular barcode information, the short-read sequence generated by the sequencer can be reassembled (restored) into the original long-fragment nucleic acid molecular sequence, thereby achieving the sequencing of full-length mRNA, full-length mitochondria, and long-length DNA.

### BRIEF DESCRIPTION OF DRAWINGS

FIG. 1 is a schematic diagram of a sequencing technology of full-length transcripts in combination with molecular barcodes according to an embodiment of the present disclosure;
FIG. 2 is a structural schematic diagram of a carrier having a molecular barcode sequence according to an embodiment of the present disclosure;
FIG. 3 is a schematic diagram of a molecular structure of an ultra-long double-stranded cDNA with a linker sequence according to an embodiment of the present disclosure;
FIG. 4 is a schematic diagram illustrating a binding of two different transposition complex structures and a full-length transcribed cDNA molecule according to an embodiment of the present disclosure;
FIG. 5 is a principle diagram of binding a carrier having a molecular barcode sequence to a transposition complex 1, transferring the molecular barcode sequence, and releasing cDNA molecules from the carrier according to an embodiment of the present disclosure;
FIG. 6 is a principle diagram of binding a carrier having a molecular barcode sequence to a transposition complex 2, transferring the molecular barcode sequence, and releasing cDNA molecules from the carrier according to an embodiment of the present disclosure;
FIG. 7 is an agarose gel electrophoresis diagram for result of a full-length transcript according to an embodiment of the present disclosure;
FIG. 8 is an agarose gel electrophoresis diagram of a double-stranded cyclization product of a full-length transcript product according to an embodiment of the present disclosure;
FIG. 9 is an agarose gel electrophoresis diagram of a cyclization product rolling circle amplification and a rolling circle amplification product two-strand synthesis of a full-length transcript product according to an embodiment of the present disclosure; and
FIG. 10 is an agarose gel electrophoresis diagram of a small fragment having barcode sequence that obtained by using transposition complex 1 according to an embodiment of the present disclosure.

### DESCRIPTION OF EMBODIMENTS

The present disclosure will be further explained in detail through specific embodiments and drawings. In the following embodiments, many details are described in order to facilitate the understanding of the present disclosure. Those skilled in the art can easily recognize that some of the features can be omitted under different circumstances, or can be replaced by other elements, materials, and methods.

In addition, the features, operations, or characteristics described in the specification may be combined in any suitable manner to form various embodiments. At the same time, the steps or actions in the method description can also be exchanged or adjusted in sequence in a manner apparent to those skilled in the art. Therefore, the various sequences in the specification and drawings are only for the purpose of clearly describing a certain embodiment, and are not meant to be a necessary sequence, unless otherwise stated that a certain sequence must be followed.

In view of the limitations and deficiencies of the current methods for constructing genomic and transcriptome libraries, the present disclosure provides a library construction method based on the preparation and enrichment of a long-fragment DNA combined with short-read sequence co-barcoding, which can solve the problem of breakage in the process of DNA fragmentation and can obtain long-fragment information without breaking DNA fragments to hundreds of bp, and reduce the loss caused by the breaking process. For highly degraded FFPE samples, the method of the present disclosure requires no breaking, and the subsequent library construction can be directly carried out, thereby greatly reducing the DNA loss in the library construction process and improving the detection efficiency. In addition, the method of the present disclosure adopts the rolling circle amplification technology to obtain the multi-copy ultra-long fragments of the circular DNA, which can solve the problem of uneven genome coverage caused by the restriction of transposase in the process of library construction due to the single copy short-fragment sequence (such as FFPE sample and mRNA full length, etc.), thereby improving the detection coverage, facilitating assembly and de novo sequencing and expanding the application range.

The method of the present disclosure labels all short-read sequences from one long-fragment DNA with the same molecular barcode, in order to obtain the original information of the long fragment. The long-fragment DNA sequence can be cDNA sequence after reverse transcription of full-length mRNA, full-length cDNA sequence of 18S rRNA or 16S rRNA, long genomic DNA fragment, full-length of mitochondrial or small genomic sequence. Therefore, the application fields of the method of the present disclosure include, but are not limited to, full-length transcript resequencing, full-length transcript assembly sequencing, full-length sequencing of 18S rRNA or 16S rRNA, full-length mitochondrial sequencing, long-fragment amplicon sequencing and the like.

The method basically includes: performing double strand cyclization of a long-fragment DNA sequence, performing rolling circle amplification to obtain continuous multi-copy ultra-long single-stranded DNA fragments of the long-fragment DNA sequence, synthesizing the double-stranded ultra-long DNA fragments by using a specific primer, labeling DNA in the same virtual compartment with the same molecular barcode by using virtual compartments and rapid enzyme reaction, and then carrying out conventional library construction and sequencing. After the sequencing, based on the molecular barcode information, the short-read sequence generated by the sequencer can be reassembled (restored) into the original long-fragment DNA sequence, thereby achieving the sequencing of full-length mRNA, full-length mitochondria, and long-fragment DNA sequences.

Compared with the prior art, the advantages of the present disclosure include at least the following aspects: (1) full-length detection of mRNA transcripts, 18S rRNA, or 16S rRNA can be performed; (2) long-fragment DNA sequences can be sequenced, thereby improving the coverage of genome detection, and facilitating the de novo sequencing of new species and genome assembly; (3) for special samples such as Paraffin-Embedded samples, or small genome samples such as mitochondria, a long-fragment library construction and sequencing can be carried out directly; and (4) a capture efficiency of the targeted sequencing target region can be improved.

The technical solutions of the present disclosure include a long-fragment DNA preparation and enrichment technology and a virtual compartment labeling technology. Specifically, (1) the technology of preparing and enriching a long-fragment DNA is to connect DNA molecules with specific linker sequences at both terminals to form a ring. Then, the circular DNA is used as template for multi-copy enrichment to obtain continuous multi-copy ultra-long DNA fragment single strand; and finally, the double strands are synthesized to finish the preparation and enrichment of long DNA fragment. (2) The theoretical principle of the virtual compartment labeling technology is that a rate of molecular thermal movement is relatively stable, and thus within a certain period of time, the range of molecular thermal movement is limited, the liquid space within a certain radius can be regarded as a "virtual" compartment. When a volume of liquid is large enough and a number of molecules is small enough, a distance between molecules is large, and two independent molecules can be regarded as completely isolated without interaction. For example, after the full-length transcript of mRNA is prepared, in a single reaction system, a carrier having a molecular barcode is added to virtually compartment DNA molecules. Finally, through the process of fragmentation and tagging, all short-read sequences from the same DNA are labeled with the same molecular barcode.

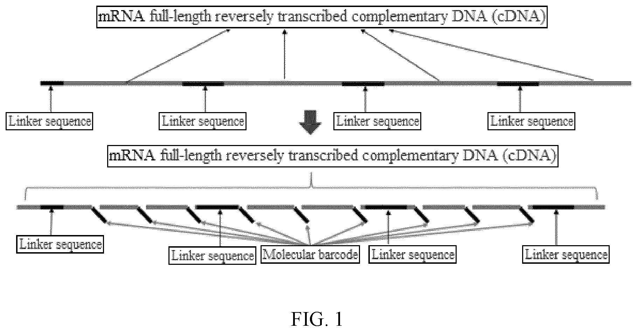

As shown in FIG. 1, a sequencing of full-length transcripts of mRNA is taken as an example, a typical but non-limiting exemplary technical solution of the present disclosure includes: firstly, extracting total RNA by conventional methods, then capturing and separating mRNA by polythymidine deoxynucleotide (oligo dT) having specific linker sequences, performing reverse transcription and synthesizing two strands to obtain a full-length cDNA molecule, and introducing the same linker sequence at the other terminal of the cDNA molecule; then, digesting the cDNA molecule having the specific linker sequences at both terminals in such a manner that both terminals become sticky terminals, and connecting the terminals of the cDNA molecule into ring using a ligase; performing multi-copy enrichment using the cyclic cDNA as a template to obtain a single strand of multi-copy ultra-long cDNA fragments, and synthesizing the second strand to obtain double-stranded cDNA ultra-long fragments, thereby completing the preparation and enrichment of mRNA full-length transcripts; after that, mixing a certain number of multi-copy ultra-long cDNA molecules with a transposase complex, and at a certain temperature, randomly inserting and binding the transposase complex to the long-fragment cDNA molecule; adding the cDNA molecule having the transposase complex into a fixed container, while adding the carrier carrying a large number of specific molecular barcodes and biochemical reagents; then, controlling a reaction volume, a concentration of cDNA molecules, a concentration of carriers, and a reaction time, so that each carrier having a large number of specific barcodes forms a virtual compartment and can capture the transposase complex bound to long-fragment cDNA molecules. When the concentration of long-fragment cDNA molecules is low enough and the number of carriers with molecular barcodes is large enough, only one cDNA molecule will be captured by one carrier, so as to form virtual compartments between the cDNA molecules falling on different carriers. After the carrier captures the cDNA molecule, the barcode on the carrier is linked with the linker sequence of the transposition complex to transfer the barcode to the cDNA fragment, and then the transposase is released to finally break a long cDNA fragment into many short fragments suitable for sequencing, and the barcodes carried by these short fragments from the same long cDNA molecule are the same. After that, the short-read long sequence generated by sequencing can be restored to the original long cDNA based on barcode information, thereby achieving the sequencing of full-length transcripts.

In a non-limiting embodiment of the present disclosure, the implementation route of the method of the present disclosure includes four parts. The first part is to prepare a large number of carriers having multi-copy specific barcode sequences (molecular barcodes); the second part is the preparation and enrichment of mRNA full-length transcripts (cDNA); the third part is to bind the enriched ultra-long DNA double-stranded molecules to the transposase complex; and the fourth part is to hybridize and capture, by the carrier having molecular barcodes, the ultra-long DNA double-stranded molecules having the transposition complex, and transfer the molecular barcodes to DNA molecules.

The above four parts will be described in detail with reference to the attached drawings as below.

### Part I

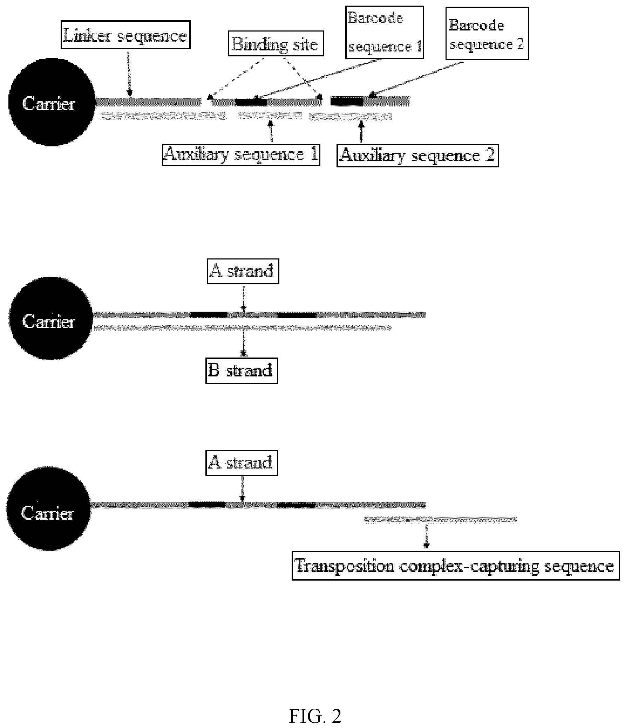

A large number of carriers having multi-copy specific barcode sequences (molecular barcodes) are prepared, that is, one carrier has multi-copy oligonucleotide sequences of the same sequence. The method adopts the technical means of "dispersion-combination-dispersion" to construct various carriers having multi-copy specific barcode sequences. As shown in FIG. 2, in a non-limiting embodiment of the present disclosure, the main flow is path as follows:
1. A specific linker sequence is linked to a carrier modified with a streptavidin protein by biotin-streptavidin interaction. In one embodiment, the carrier can be an iron oxide magnetic bead carrier, and in other embodiments, it can be other solid-phase carriers such as cross-linked agarose, agar, polystyrene, polyacrylamide, glass, etc., whose surface is modified with streptavidin to facilitate biotin modification and binding on a specific linker sequence. In other embodiments, the surface modification of the solid carrier can be any molecule that can cross-link the DNA oligonucleotide (i.e., a linker sequence), such as hydroxyl, carboxyl, amino, etc.
2. A barcode sequence 1 and an auxiliary sequence 1 with different numbers (No.1-1536) are distributed in different wells of 384-well plates, and annealed, totaling four 384-well plates. The barcode sequence 1 is divided into three parts. The first part is a sequence complementary to an antisense strand of the specific linker sequence and complementary to the auxiliary sequence 1, composed of 4-50 bases. In a preferrable embodiment, the sequence complementary to the antisense strand of the specific linker sequence and the sequence complementary to the auxiliary sequence 1 are 6 and 15 bases, respectively. The second part is the specific molecular barcode sequence, composed of 4-50 bases, and in a preferrable embodiment, it is composed of 10 bases. The third part is 4-50 bases complementary to the auxiliary sequence 2, and in a preferrable embodiment, it has 6 bases. The auxiliary sequence 1 is composed of a sequence partially complementary to barcode sequence 1 and can be 4-50 bases, and in a preferrable embodiment, it is 21 bases. In a preferrable embodiment, the barcode sequence 1 and the auxiliary sequence 1 are composed of four bases of A, T, C, and G, and each base cannot successively repeat more than three times. After annealing, a partially double-stranded sticky terminal structure is formed, which is convenient for connecting with a carrier having specific linker sequence and connecting with the annealed barcode sequence 2. It should be noted that the structure and composition of the barcode sequence 1 described above is only an example, and the base composition and number of each part can be arbitrarily changed according to specific needs. In addition, the connection mode of the barcode sequence can be that terminal 3' of the sequence is connected to the carrier magnetic bead, or terminal 5' of the sequence is connected to the carrier of magnetic bead. The barcode sequence can be either a single-stranded sequence or a double-stranded sequence.
3. The carriers having linker sequences in step 1 are evenly distributed to each well of the four 384-well plates. DNA ligase is used to connect the linker sequence on the carrier to the annealed barcode sequence 1. The barcode sequence 1 contains a specific DNA sequence, which is molecular barcode 1.
4. A large amount of buffer solution is used to wash the carriers, in order to remove the ligase in the previous step and the oligonucleotide that failed to react completely.
5. The carriers washed in step 4 are collected by centrifugation, and uniformly mixed with an oscillating mixer.
6. A barcode sequence 2 and an auxiliary sequence 2 with different numbers (No.1-1536) are added in different well of brand-new 384-well plates and annealed, totally four 384-well plates. After that, the carriers evenly mixed in step 5 are evenly distributed to each well. The barcode sequence 2 consists of a specific molecular barcode sequence, a sequence complementary to the auxiliary sequence 2, and a sequence complementary to the transposition complex-capturing sequence. These three sequences can be 4-50 bases, respectively, and in a preferrable embodiment, they are 10, 10 and 15 bases respectively. The auxiliary sequence 2 is composed of a sequence partially complementary to barcode sequence 1 and a sequence partially complementary to the barcode sequence 2. These two sequences can be 4-50 bases, respectively, and in a preferrable embodiment, they are 6 and 20 bases, respectively. In a preferrable embodiment, the barcode sequence 2 and the auxiliary sequence 2 are composed of four bases of A, T, C, and G, and each base cannot successively repeat for more than three times. After annealing, a partially double-stranded sticky terminal structure is formed, which is convenient for connecting with a carrier having a specific linker sequence and the annealed barcode sequence 1.
7. DNA ligase is used to link the barcode sequence 1 in the carrier to the barcode sequence 2. The barcode sequence 2 contains a specific DNA sequence, which is molecular barcode 2.
8. A large amount of buffer solution is used to wash the carriers, in order to remove the ligase in the previous step and the oligonucleotide that failed to react completely.
9. After the above preparation, a carrier containing partially double-stranded two barcode sequences is obtained; the DNA sequence containing two barcode sequences is A strand, and the complementary strand thereof is B strand.
10. The B strand on the carrier is denatured, and then the carrier is washed with a buffer solution and then annealed with a transposase complex-capturing sequence.
11. A large amount of buffer solution is used to wash the carriers, in order to remove the oligonucleotides which fail to react completely in the previous step. At this point, the preparation of 1536*1536 molecular barcode carriers (i.e., 2,359,296 types) of the scheme 1 is completed.
12. In the carrier preparation scheme 2, after the above steps are completed, the annealed transposon 1 (for example, referring to as transposon 1 in the following example) and the DNA sequence on the carrier in step 11 are connected using DNA ligase.
13. A large amount of buffer solution is used to wash the carriers, in order to remove the oligonucleotides which fail to react completely in the previous step. So far, the preparation of 1536*1536 molecular barcode carriers in the scheme 2 is completed.

It should be noted that the above description of the first part is merely illustrative. In particular, the number of molecular barcodes is not limited to the above 2,359,296, but can be increased or decreased, but at least cannot be less than 2. For example, in other embodiments, only a single barcode sequence numbered 1-1536 may be used instead of the combination of barcode sequence 1 and barcode sequence 2 described above. In other embodiments, it is also possible to use combinations of three or more barcode sequences, for example, each of barcode sequence 1, barcode sequence 2, and barcode sequence 3 has numbers 1-1536 respectively, and thus there are 1536*1536*1536 molecular barcode carriers.

### Part II

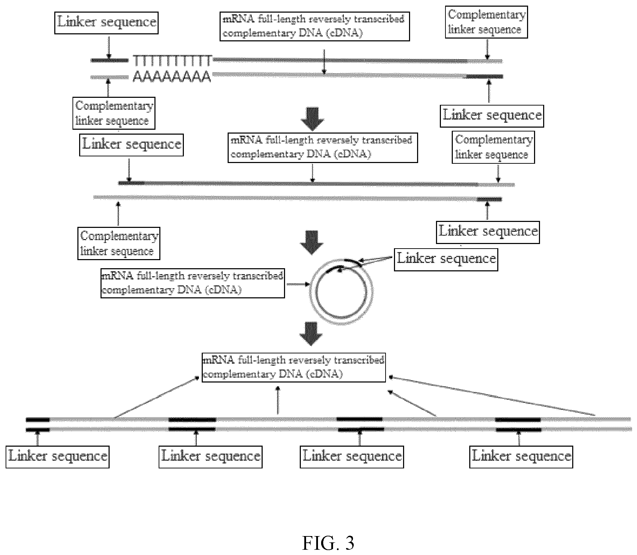

A sequencing of mRNA full-length transcripts is taken as an example, the preparation and enrichment of mRNA full-length transcripts (cDNA) refers to linking multi-copy cDNA full-length sequences together. In an embodiment of the present disclosure, the full-length cDNA is prepared and enriched by adopting the technical means of double-strand cyclization and rolling circle amplification. As shown in FIG. 3, in a non-limiting embodiment of the present disclosure, the main flow path is as follows:
1. The full-length mRNA is captured by polythymidine deoxynucleotide (oligo dT) having a linker sequence and then subjected to reverse transcription. By using the terminal transferase activity of reverse transcriptase, the same linker sequence is introduced to the other terminal of the cDNA molecule while synthesizing the second strand, and the full-length cDNA molecule is obtained by one-step extension. The linker sequence has a U base, which can be cleaved by USER enzyme. In other embodiments, instead of U base sites, the linker sequence carries other types of cleavage sites and forms sticky terminals at both terminals of the cDNA molecule through the digestion of corresponding enzymes. For example, the I base is cleaved by endo V enzyme to form sticky terminals.
2. The U base in the linker sequence at both terminals of cDNA molecule is excised by USER enzyme, for forming sticky terminals at both terminals of cDNA molecule. In other embodiments, the linker sequence has restriction sites, and the sticky terminal is formed by restriction enzyme digestion.
3. Using DNA ligase, the palindromic sequences at the sticky terminals of cDNA molecules are connected end to end to form double-stranded cyclic cDNA molecules.
4. Using the double-stranded cyclic cDNA molecules as a template and oligo dT as a primer, multi-copy cDNA molecules are enriched by phi29 DNA polymerase, and the continuous multi-copy ultra-long single-stranded cDNA molecules are obtained.
5. Using the ultra-long single-stranded cDNA molecules as a template and the fragment of the linker sequence as a primer, the DNA polymerase I and DNA ligase are used to synthesize complementary double strands, and an ultra-long double-stranded cDNA molecule is obtained, thereby completing the preparation and enrichment of mRNA full-length transcript (cDNA) .

It should be noted that the enzymes used in the preparation and enrichment of mRNA full-length transcripts (cDNA) are not limited to the above-mentioned phi29 DNA polymerase, DNA polymerase I, and DNA ligase, etc., but can be replaced by other enzymes with the same functions. In addition, during the preparation and enrichment of mRNA full-length transcripts (cDNA), the reaction system used can be adjusted according to the input amount of reactants, and the enzyme amount used in the reaction system can also be adjusted according to the input amount of reactants.

### Part III

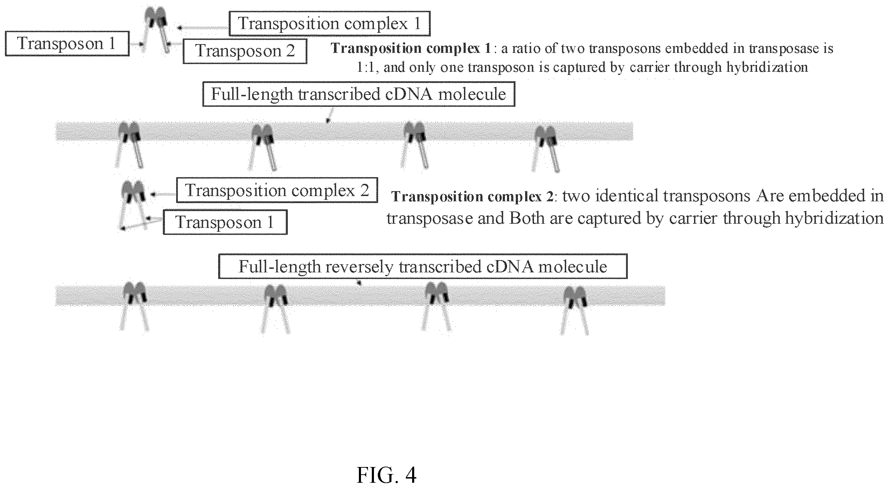

Transposase, as a commonly used tool enzyme for library construction, has the advantages of fast reaction speed and one-step fragmentation and labeling, etc. At the same time, the transposase also has the characteristic that after the transposition reaction, the DNA fragment can be kept intact without denaturation treatment. Therefore, in the embodiment of the present disclosure, the transposase is used to fragment high molecular weight DNA. As shown in FIG. 4, in a non-limiting embodiment of the present disclosure, the specific flow path is as follows:
1. A transposon sequence is mixed with a transposase at 30°C and incubated for one hour at 30°C to form a transposition complex, which is taken out and placed in a refrigerator at -20°C for use. In one embodiment, the transposase is a Tn5 transposase. In other embodiments, it can be other enzymes of Tn transposase family, such as Tn7, or other transposase families, such as the Mu family; it is even not limited to a transposase or an enzyme preparation, as long as it can fragment cDNA and connect a sequence to the cDNA.
2. Then a certain amount of transposition complexes is incubated with the DNA of a high molecular weight at 55°C for 10 minutes.
3. No transposase is released, leaving the cDNA molecules intact.

As shown in FIG. 4, two types of transposition complexes can be used in the present disclosure. One of them is transposition complex 1, the transposase embeds two types of transposons, namely transposon 1 and transposon 2, and only one type of transposon such as transposon 1 can be captured by carrier through hybridization. In one embodiment, each transposon sequence includes a sense strand and an antisense strand, and the sense strand of transposon 1 in transposition complex 1 is connected to the molecular barcode sequence, while the sense strand of transposon 2 is not connected to the molecular barcode sequence; or vice versa. And the antisense strand has a U base site, which can be cleaved by USER enzyme digestion, thereby facilitating the subsequent PCR amplification.

The other one is transposition complex 2, in which two identical transposons (e.g., transposon 1) are embedded in the transposase and can be captured by the carrier through hybridization. In an embodiment, each transposon sequence includes a sense strand and an antisense strand, and the sense strand of each transposon sequence is connected to a molecular barcode sequence, and the antisense strand has a U base site, which can be cleaved by USER enzyme digestion.

It should be noted that the method of digesting the antisense strand on transposon is not limited to cleaving the U base site by using the USER/UDG&APE1-combined enzyme digestion method, but it can also use exonuclease III, Lambda exonuclease or other enzymes or reagents that can specifically or non-specifically digest the antisense strand. The position and number of the U bases for replacing T bases on the antisense strand of transposon are not limited, and any T bases on the sequence can be replaced. In addition, that base is not limit to U base, but can be other specially modified bases, such as methylated base, and the position and the number of the replacement bases are not limited, and any base on the sequence can be replaced. In addition, in the embodiments of the present disclosure, the length and sequence information of the transposon sequence are not limited.

### Part IV

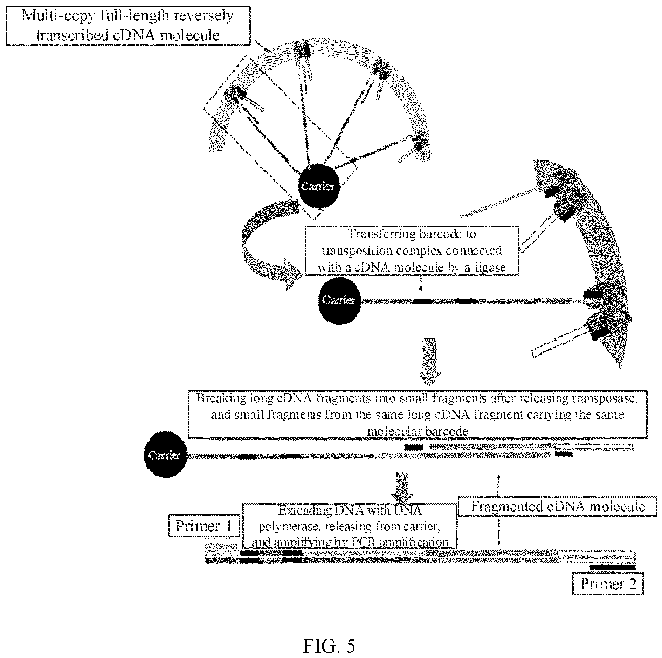

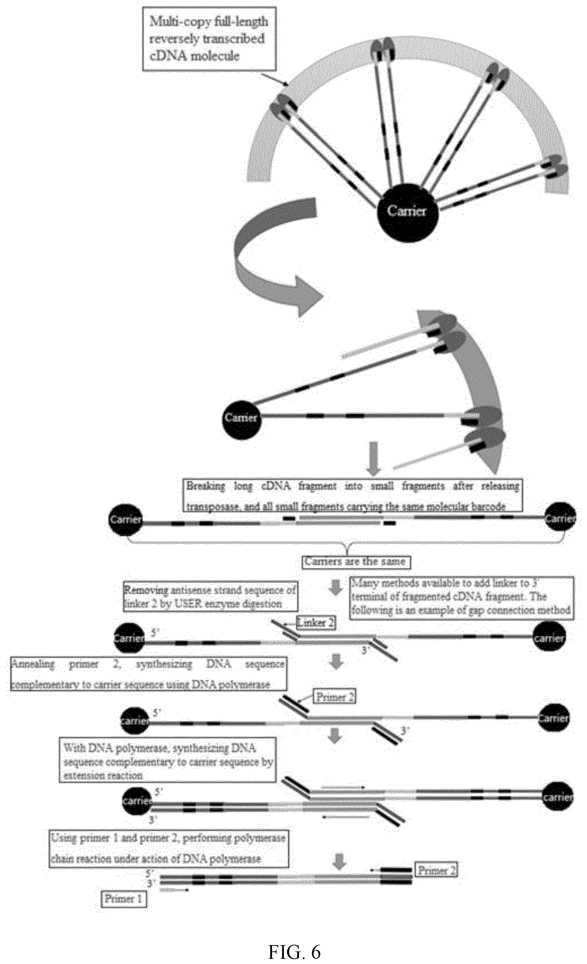

A carrier having a molecular barcode is combined with a transposase complex, transferring the barcode and releasing the cDNA molecule from the carrier, as shown in FIG. 5 and FIG. 6, the subsequent treatment methods are different depending on the type of the transposase complex used.
1. The long-fragment cDNA having transposase complex is diluted and then mixed with the carrier having the barcode, the carrier will capture the transposase complex in the way of cDNA sequence hybridization. The amount of carriers having the barcode can be specifically adjusted and determined according to the input amount of reactants. In one embodiment, the amount of carriers having the barcode is tens of thousands, hundreds of thousands, millions, tens of millions, or even hundreds of millions. In other embodiments, the amount of carriers having the barcode can be increased or decreased as appropriate.
2. When the transposition complex 1 is used, DNA ligase connects the carrier sequence having the barcode to the transposition complex of DNA molecule, i.e., the barcode is transferred to the transposition complex connected to cDNA molecule by the ligase.
3. When the transposition complex 1 is used, after the transposase is released, the long-fragment cDNA molecules are broken into small fragments, and the small fragments from the same long-fragment cDNA molecule all carried the same molecular barcode. The USER enzyme cleaves the sequence of the transposase, then a DNA polymerase is used to carry out extension reaction to release DNA from the carrier, and then a partial sequence of the specific linker sequence is used as a primer 1 and a partial sequence of the sense strand of the transposon 2 as a primer 2 to carry out DNA molecular polymerase chain amplification, so as to obtain short-fragment molecules having molecular barcodes suitable for sequencing.
4. When transposition complex 2 is used, after transposase is released, long-fragment cDNA molecules are broken into small fragments, and all small fragments from the same long-fragment cDNA molecule are labeled with the same molecular barcode. The linker 2 is connected to the gap by a ligase, as shown in FIG. 6. In one embodiment of the present disclosure, the linker 2 is connected to the gap by gap ligation method, then using the sequence complementary to the sense strand of the linker 2 as a primer 2, the DNA sequence complementary to the carrier sequence is synthesized by extension reaction under the action of the DNA polymerase.
   It should be noted that there are many methods to add a linker to the 3' terminal of the fragmented cDNA fragment, such as poly C/A/T/G base tail strand transfer, extension termination strand transfer, asymmetric linker gap filling, single strand random sequence filling, etc.
5. The partial sequence of the specific linker sequences is used as a primer 1, and the sequence complementary to the sense strand of the linker 2 is used as a primer to carry out polymerase chain amplification of DNA molecules, so as to obtain short-fragment molecules having molecular barcodes suitable for sequencing.
6. A suitable sequencing platform is used for sequencing, and based on the molecular barcode information, the short-fragment information obtained by sequencing can be restored to the long-fragment information of cDNA, so as to obtain the total mRNA expression in cells.

Taking the sequencing of mRNA full-length transcripts as an example, the method of the present disclosure provides a solution for sequencing mRNA full-length transcripts, which successfully solves the problems of information loss caused by fragmentation in short-read sequencing methods and incapability of measuring the full length. The method of the present disclosure can improve the capture efficiency of the target region in targeted sequencing. The sequencing data generated by the method for constructing a library according to the present disclosure can be used for de novo assembly of genome or transcriptome.

The technical solution and effects of the present disclosure will be explained in detail below through specific examples. It should be understood that the examples are only exemplary and cannot be understood as limiting the scope of protection of the present disclosure.

### Examples

### Part I: preparing a large number of carriers having multi-copy molecular barcode sequences

1. A specific linker sequence was linked to a carrier modified with a streptavidin protein by biotin-streptavidin interaction. In this embodiment, the specific linker was a double-stranded DNA molecule, which was annealed together by two single-stranded DNA strands.
   The sense strand of the linker sequence was Linker-F (5'-2-bio-AAAAAAAAAATGTGAGCCAAGGAGTTG-3', modified with a double biotin at 5'-terminal, SEQ ID NO: 1); and the antisense strand of the linker sequence was Linker-R (5'-CCAGAGCAACTCCTTGGCTCACA-3', SEQ ID NO:2). The annealing conditions of the two single-stranded DNAs were 70°C for 1 minute, then the temperature was slowly lowered to 20°C at a speed of 0.1°C/s, and the reaction was carried out at 20°C for 30 minutes. The magnetic beads with streptavidin were Dynabeads M-280 streptation (112.06D, streptavidin immunomagnetic bead, Invitrogen). Linker (50µM) and M-280 magnetic beads were mixed at a ratio of 2µL to 30µL, the preservation solution of magnetic beads was replaced with 1-fold concentration of a magnetic bead binding buffer (50mM Tirs-HCl, 150mM NaCl, 0.1mM EDTA), and then mixed on a vertical mixer at 25°C for 1 hour, and then washed with low-salt magnetic bead buffer (50mM Tirs-HCl, 150mM NaCl, 0.02% Tween-20) twice, and finally the magnetic beads were resuspended with 12.5µL (2µL of Linker + 30µL of magnetic beads, a concentration of Linker was 1.6µM) of 1-fold concentration of ligation buffer (a working solution concentration of ligation buffer was 3-fold, PEG8000 30%, Tris-HCl 150mM, ATP 1mM, BSA 0.15mg/mL, MgCl₂ 30mM, DTT 1.5mM).
2. Barcode sequence 1 and auxiliary sequence 1 with different numbers (No.1-1536) were distributed in different wells of 384-well plates for annealing (1:1 annealing, 4µL/well), totally four 384-well plates.
   Barcode sequence 1: 5'Phos-CTCTGGCGACGGCCACGAAGC[Barcode]TCTGCG-3' (SEQ ID NO: 3);
   Auxiliary sequence 1: 5'-[Barcode]GCTTCGTGGCCGTCG-3' (SEQ ID NO: 4).
   Barcode represents a barcode sequence randomly synthesized by the instrument, for example, 10 random bases N, where N can be any one of A, T, G and C.
   The annealing conditions of barcode sequence 1 and auxiliary sequence 1 were as follows: barcode sequence 1 (100µM) and auxiliary sequence 1 were mixed at a ratio of 1:1, and then placed on a PCR instrument at 70°C for 1 minute, then cooled slowly to 20°C at a speed of 0.1°C/s, and reacted for 30 minutes at 20°C.
3. DNA ligase was used to connect the linker sequence on the carrier having barcode sequence 1 to the barcode sequence 1. The barcode sequence 1 contains a specific DNA sequence as molecular barcode 1. The specific steps were as follows: M280 magnetic beads with Linker in step 1 were evenly distributed to 1536 wells of the four 384-well plates, 2.5µL per well. Then, 3.5µL of 3-fold concentration of ligase buffer mixture (1µL of T4 DNA ligase (600U/µL), and 2.5µL of a ligation buffer) was added to each well, and the ligation reaction was carried out at 25°C for 1 hour under a condition of a total reaction volume of 10µL in each 384-well plate.
4. A large amount of high-salt magnetic bead washing buffer (50mM Tirs-HCl, 500mM NaCl, and 0.02% Tween-20) was used for once washing, and then a large amount of low-salt magnetic bead washing buffer (50mM Tirs-HCl, 150mM NaCl, and 0.02% Tween-20) was used for once washing, in order to remove ligases and oligonucleotides that did not react completely in the previous step.
5. The magnetic beads washed in step 4 were collected through a magnetic rack, and then resuspended with 1-fold concentration of ligation buffer; after a concentration of the resuspended Linkers was 1.6µM, and they were mixed uniformly with an oscillating mixer.
6. The barcode sequence 2 and auxiliary sequence 2 with different numbers (No.1-1536) were annealed in different wells of brand-new 384-well plates, totally four 384-well plates.
   Barcode sequence 2: 5'-[Barcode]TAGCATGGACTATGG-3' (SEQ ID NO:5);
   Auxiliary sequence 2: 5'-GTCCATGCTA[Barcode]CGCAGA-3' (SEQ ID NO:6).
   Barcode represents a barcode sequence randomly synthesized by the instrument, for example, 10 random bases N, where N can be any one of A, T, G and C.
   The annealing conditions of barcode sequence 2 and auxiliary sequence 2 were as follows: 2µL of barcode sequence 2 (100µM) and 2µL of auxiliary sequence 2 (100µM) were mixed in a 384-well plate, which was then placed on a PCR instrument at 70°C for 1 minute, then cooled slowly to 20°C at a speed of 0.1°C/s, and reacted for 30 minutes at 20°C.
7. The magnetic beads evenly mixed in step 5 were distributed to each well of the 384-well plates in step 6 in an amount of 2.5µL per well. Then, 3.5µL of a mixture of the ligase buffer (1µL of T4 DNA ligase (600U/µL), and 2.5µL of ligation buffer) was added and reacted at 25°C for 1 hour.
8. A large amount of high-salt magnetic bead washing buffer (50mM Tirs-HCl, 500mM NaCl, and 0.02% Tween-20) was used for once washing, and then a large amount of low-salt magnetic bead washing buffer (50mM Tirs-HCl, 150mM NaCl, and 0.02% Tween-20) were used for once washing, in order to remove ligases and oligonucleotides that did not react completely in the previous reaction.
9. 1 billion magnetic beads were taken from each portion, the low-salt magnetic bead washing solution was removed from the magnetic beads with a magnetic rack, washed once with 50µL of a low-salt magnetic bead washing solution, then resuspended with 50µL of a strong alkali denaturation buffer (KOH 1.6M, EDTA 1 mm), incubated at room temperature for 5 minutes, then absorbed with a magnetic rack for 2 minutes to remove the strong alkali denaturation buffer, and then 50µL of the strong alkali denaturation buffer was added to wash the magnetic beads once; 10µL of an annealing buffer (400 mM Tris-HCl, 500 mM NaCl, 100 mM MgCl₂, pH 7.9) was added after removing the strong alkali denaturation buffer, 5.7µL of transposition complex-capturing sequence (100µM) was diluted with water to a volume of 100µL. The sequence of the capture transposition complex was AUCGUACCUGAUACCGCUAGGAACCACUAGUACAGCAGUCACG (SEQ ID NO: 7), then annealed at 60°C for 5 minutes, and reacted 25°C for 1 hour.
10. The oligonucleotides that did not react completely in the previous step were removed, the magnetic beads were collected with a magnetic rack, washed with a low-salt buffer, and finally resuspended in the low-salt magnetic bead washing buffer, and can be stored at 4°C for one year.
11. At this step, 2,359,296 types of molecular label magnetic bead carriers of the scheme 1 were prepared.
12. In the scheme 2 of carrier preparation: the barcode magnetic bead carriers prepared in step 10 were placed on a magnetic rack, after removing the low-salt buffer, 11.4µL of transposon 1 (50µM) was added, 45µL of a 3-fold concentration of a ligase buffer mixture (12µL of T4 DNA ligase (600U/µL), 33µL of a 3-fold concentration of a ligase buffer) was added, diluted with water to a volume of 100µL, and reacted at 25°C for 1 hour.
13. A large amount of high-salt magnetic bead washing buffer (50mM Tirs-HCl, 500mM NaCl, and 0.02% Tween-20) was used for once washing, and then a large amount of low-salt magnetic bead washing buffer (50mM Tirs-HCl, 150mM NaCl, and 0.02% Tween-20) were used for once washing, in order to remove ligases and oligonucleotides which did not react completely in the previous step. The magnetic beads were collected by a magnetic rack, and then resuspended in a low-salt magnetic bead washing buffer, and can be stored at 4°C for 1 year.
14. At this step, in the scheme 2, 2,359,296 types of molecular label magnetic bead carriers were prepared.

### Part II: preparation of a transposition complex

1. Preparation of a transposon: the transposon was formed by annealing two DNA single-stranded molecules. Transposon 1 and transposon 2 were included in transposition complex 1 and they were different from each other. The sense strand of the transposon 1 constituting the transposition complex 1 was a transposon 1-F, and the antisense strand of the transposon 1 constituting the transposition complex 1 was a transposon 1-R. The sense strand of the transposon 2 constituting the transposition complex 1 was a transposon 2-F, and the antisense strand of the transposon 2 constituting the transposition complex 1 was a transposon 2-R.
   Transposon 1-F: 5'phos-CGATCCTTGGTGATCATGTCGTCAGTGCTTGTCTTCCTAAGATGTGTATAAGAGACAG-3' (SEQ ID NO:8);
   Transposon 1-R: 5'phos-CTGTCTCUTATACACATCT-3' (SEQ ID NO: 9);
   Transposon 2-F: 5'-GAGACGTTCTCGACTCAGCAGAAGATGTGTATAAGAGACAG-3' (SEQ ID NO: 10);
   Transposon 2-R: 5'Phos-CTGTCTCUTATACACATCT-3' (SEQ ID NO:11).
      Two identical transposons 1 were included in transposition complex 2. The sense strand of the transposon 1 constituting the transposition complex 2 was transposon 1-F, and the antisense strand of the transposon 1 constituting the transposition complex 2 was transposon 1-R.
      The annealing conditions were as follows: 20µL of the sense strand of transposon and 20µL of antisense strand, at a concentration of 100µM, were mixed with each other at 70°C for 1 minute, then slowly cooled to 20°C at a speed of 0.1°C/s, and reacted at 20°C for 30 minutes, to finally obtain the transposon with a concentration of 50µM.
2. When applying the magnetic bead carriers according to the scheme 1 in Part I, 11.8µL of Tn5 transposase (1U/µL) within the shelf life, 1.6µL of the transposon 1 and 1.6µL of the transposon 2 prepared in the previous step or 3.2µL of the transposon 1, and 25µL of 50% glycerol diluted with a TE buffer (10mM Tris-HCl, and 1mM EDTA) were mixed on ice and then reacted at 30°C for 1 hour; after the reaction was completed, the product was transposition complex 1 or transposition complex 2, the transposon concentration in the transposition complex was 4pmol/µL, and the prepared transposition complex could be stored at -20°C for one year.
3. When applying the magnetic bead carriers according to the scheme 2 in Part I and using transposition complex 1, 5.3µL of the magnetic bead carriers prepared according to the scheme 2 in Part I was taken, washed twice with a low-salt magnetic bead washing buffer, 0.6µL of a double-stranded transposon 2 annealed in step 1) was added, and 4.3µL of the TN5 transposase (1U/µL) was embedded in 10.1µL of an embedding reaction solution (50% glycerol; 50% TE), while being placed on a vertical mixer, and incubated at 30°C for 1 hour; after embedding, a final concentration of transposon was 4pmol/µL.
4. When applying the magnetic bead carriers according to the scheme 2 in Part I and only using the transposition complex 2, 5.3µL of the magnetic bead carriers prepared according to the scheme 2 in Part I was taken, washed twice with a low-salt magnetic bead washing buffer, and 4.3µL of the Tn5 transposase (1U/µL) was added and embedded in 10.7µL of an embedding reaction solution (50% glycerol; 50% TE), while being placed on a vertical mixer during embedding, and incubated at 30°C for 1 hour; after embedding, a final concentration of transposon is 4pmol/µL.

### Part III: Preparation and enrichment of mRNA full-length transcript (cDNA)

The preparation and enrichment of mRNA full-length transcripts (cDNA) refers to connecting multi-copy cDNA full-length sequences together. In this example, the full-length cDNA was prepared and enriched by double-strand cyclization and rolling circle amplification.
1. The capturing sequence for capturing mRNA, TSO primer and ISO primer for reverse transcription, oligo dT sequence for rolling circle amplification, and Tn primer for synthesizing double strands were synthesized in advance, all of which were dissolved with a TE solution to a concentration of 100µM and stored at -20°C for use. In this example, 1µg of RNA in total was used.
   Capturing sequence: 5'-AAGCdUdUCGTAGCCATGTCGTTCTGCGNNNNNNNNNNTTTTTTTTTTTTTTTTTTTTTV-3' (SEQ ID NO:12);
   TSO primer: 5'-AAGCdUdUCGTAGCCATGTCGTTCTGrGrG+G-3' (SEQ ID NO:13);
   ISO primer: 5'-AAGCdUdUCGTAGCCATGTCGTTCTG -3' (SEQ ID NO: 14);
   oligo dT sequence: 5'-TTTTTTTTTTTTTTTT-3' (SEQ ID NO:15); and
   Tn primer: 5'-CGTAGCCATGTCGTTCTG-3' (SEQ ID NO: 16) .
2. 1µL of RNA (1µg), 5µL of dNTP (10mM), and 1µL of capturing sequence (50µM) were added, placed in a PCR instrument at 72°C for 3 minutes, and immediately taken out and placed on ice for 1 minute. Then, a reverse transcriptase reaction mixture was added. The reverse transcriptase reaction mixture contained 1µL of a reverse transcriptase (SuperScript II reverse transcriptase (200U/µL), Invitrogen), 0.5µL of RNaseOUT™ (RNA enzyme inhibitor, 40U/µL, Invitrogen), 4µL of 5XSuperscript II first-strand buffer (5-fold concentration reverse transcriptase II buffer, 250 mM Tris-HCl, pH 8.3, 375 mM KCl, 15 mM MgCl₂, Invitrogen), 0.5µL of DTT(100 mM, Invitrogen), 6µL of MgCl₂ (25 mM, Invitrogen), 0.5µL of TSO primer (100µM), diluted with water to a volume of 20µL in total. The mixture was placed in a PCR instrument and the following procedures were executed: (1) 42°C for 90 minutes; (2) 50°C for 2 minutes; (3) 42°C for 2 minutes; and (2) to (3) were operated for 10 cycles.
3. After the reaction was completed, the full-length transcript amplification reaction mixture was added, including 50 µL of 2X KAPA HiFi HotStart Ready Mix (2-fold concentration of KAPA HIFI hot starter enzyme mixture) (5mM MgCl₂, 0.6mM of each dNTP, 1U KAPA HiFi HotStart DNA polymerase (1 unit of KAPAHiFi hot start DNA polymerase), KAPA), 5µL of an ISO primer (10µM), and the volume was supplemented to 100µL with water. The reaction was carried out according to the following procedures: (1) 98°C for 3 minutes; (2) 98°C for 20 seconds; (3) 67°C for 15 seconds; (4) 72°C for 6 minutes; (5) 72°C for 5 minutes; steps (2) to (5) were repeated for 1-2 cycles. It should be noted that the number of amplification cycles is related to the total RNA input. When the total RNA input is reduced, the number of amplification cycles needs to be increased. For example, when the total RNA input is lOng or 100ng, a number of amplification cycles may be 18-20 or 10-15 cycles.
4. After the reaction was completed, the above products were purified with 200µL of XP magnetic beads (Agencourt AMPure XP-Medium, A63882, AGENCOUR). The purification method can be found in the official standard operating procedures.
5. 1µL of a USER enzyme (1U/µL NEB), 3µL of 10X stTaq buffer (10-fold concentration of standard Taq buffer, 100mM Tris-HCl, 500mM KCl, 15mM MgCl₂) were added to the above product, and diluted with water to a volume of 30µL, and then placed in a PCR instrument to react at 37°C for 1 hour.
6. Immediately After the reaction was completed, the product was taken out, 5µL of 10×TA Buffer was added and diluted with water to a volume of 50µL; then the mixture was placed in PCR instrument to react at 70°C for 30 minutes, and subjected to water bath at room temperature for 20 minutes.
7. After the reaction was completed, 2.75µL of 20 Circ Mix (10×TA buffer (10-fold concentration of TA buffer), 0.1M ATP) and 0.1µL of T4 DNA ligase (600U/µL from Enzymatics) were added to the above product, and diluted with water to a volume of 55µL, and reacted at room temperature for 2 hours.
8. After the reaction was completed, the above product was purified with 55µL of XP magnetic beads (Agencourt AMPure XP-Medium, A63882, AGENCOURT), and the purification method was conducted according to the official standard operation instruction.
9. After the purification, 3µL of 10×Plasma-safe Buffer (10-fold concentration of a linear buffer) (330mM Tris-acetate (Tris-acetic acid, pH 7.5), 660mM potassium acetate, 100mM magnesium acetate, and 5.0mM DTT), 3.38µL of Plasma-Safe ATP-Dependent DNase (10U/µL, Epicentre), 1.2µL of ATP (25mM) were added to the above product, diluted with water to a volume of 30µL, and placed in a PCR instrument to react at 37°C for 1.5 hours.
10. After the reaction was completed, the above product was purified with 30µL of XP magnetic beads (Agencourt AMPure XP-Medium, A63882, AGENCOURT), and the purification method was conducted according to the official standard operation instruction. So far, double-strand cyclization of full-length transcripts has been completed.
11. Preparation of a rolling circle amplification reaction solution; 4µL of oligo dT (50µM) was taken, 40µL of 10×phi29 buffer (10-fold concentration of phi29 buffer) was added and diluted with water to a volume of 200µL, and then stored in a refrigerator at -20°C for use.
12. 20µL of the rolling circle amplification reaction solution prepared in step 11 was added to the product in step 10, and then diluted with water to a volume of 40µL. The following procedures were performed: 95°C for 1 minute, 65°C for 1 minute, and 40°C for 1 minute. After the procedures were finished, the product was taken out and placed on ice immediately.
13. 40µL of an Enzyme mixture and 4µL of an Enzyme mixture II were added to the above product, and the mixture was placed in a PCR instrument at 30°C for 10 minutes and 65°C for 10 minutes. It can be stored for one week at 4°C.
14. After the reaction was completed, the concentration was detected with a single-strand concentration detection kit (Lifetech). 100ng of the product was taken for subsequent reaction.
15. To 100ng of the product from step 13, 5µL of 10×NEB buffer 2 (10-fold concentration of NEB buffer 2), 0.4µL of dNTP Mix (25 mM each), 0.5µL of ATP (0.1M), and 0.5µL of Tn primer (10µM) were added, and the mixture was placed in a PCR instrument to run the following procedures: 95°C for 3 minutes and 58°C for 30 seconds. After the reaction was completed, the mixture was taken out immediately and added with 2µL of DNA polymerase 1 (NEB, 5U/µL) and 1µL of T4 DNA ligase (Enzymatics, 600U/µL), and the mixture was placed in a PCR instrument to perform the following procedures: 37°C for 30 minutes and 75°C for 20 minutes.
16. After the reaction was completed, the above product was purified with 50µL of XP magnetic beads (Agencourt AMPure XP-Medium, A63882, AGENCOURT), and the purification method was conducted according to the official standard operation instruction. The purified product can be stored at 4°C for one week. At this point, the preparation and enrichment of mRNA full-length transcripts (cDNA) was completed.

### Part IV: Preparation of short-fragment molecules having molecular barcodes suitable for sequencing

1. Preparation of magnetic beads with barcodes: 10 million of magnetic beads were taken, the washing solution of low-salt magnetic beads was removed by a magnetic rack, and washed with 50µL of a low-salt magnetic bead washing buffer and 50µL of a hybridization buffer (Tris-HCl 50 mM, NaCl 1000mM, Tween-20 0.05%), respectively; finally, the magnetic beads were resuspended with 50µL of 2-fold concentration of the hybridization buffer (Tris-HCl 100mM, NaCl 2000mM, and Tween-20 1%).
2. The mixed solution of transposition complex and long-fragment DNA molecule was prepared on ice. 10µL of 5-fold concentration of a transposase buffer (HEPES-KOH 50mM (potassium hydroxide), DMF 50% (dimethylformamide), and MgCl₂ 25mM (magnesium chloride)), and lOng of the long-fragment DNA molecule (i.e., the product prepared in the part III) were diluted to 1µL of the transposition complex containing 0.5pmol/µL of the transposon, and the system was diluted with water of molecular reaction grade to a volume of 50µL.
3. When using the carrier magnetic beads of the scheme 1, 50µL of the long-fragment DNA solution with transposase complex prepared in Step 2 and the 50µL of the magnetic beads having barcodes prepared in the previous step were mixed and reacted at 60°C for 1 minute, and then the reaction solution was placed at room temperature, naturally cooled, placed in a vertical mixer and mixed and reacted at 25°C for 1 hour.
4. When using transposition complex 1, after the hybridization time of 1 hour was finished, the mixed reaction solution of ligase was added to re-suspend the magnetic beads, and reacted at 20°C for 1 hour, in which the ligase (T4 DNA ligase, Enzymatics, 600 u/µL) was 1µL, the ligation buffer with of 10-fold concentration was 20µL, and the volume was increased to 200µL with water of molecular reaction grade. After the reaction was completed, 5µL of 0.44% SDS was added and incubated at room temperature for 10 minutes to denature transposase and release transposase from DNA, and then washed with high-salt magnetic bead washing solution and low-salt magnetic bead washing solution, respectively. 2µL of the USER enzyme (1U/µL NEB) was added and placed on a vertical mixer to react at 37°C for 0.5 hours, and then washed respectively with high-salt magnetic bead washing solution and low-salt magnetic bead washing buffer that were preheated at 37°C. Then the mixed solution of a polymerase reagent was added to re-suspend the magnetic beads, and reacted at 72°C for 10 minutes. Polymerase (Standard Taq polymerase, 5U/µL, NEB) was 1µL, 10xthermopol buffer (10-fold concentration of thermopol buffer, NEB, 200mM Tris-HCl, 100mM (NH₄)₂SO₄, 100mMKCl (potassium chloride), 20mM MgSO₄ (magnesium sulfate), and 1% Triton®X-100) was 5µL, 25mM dNTP (Enzymatics) was 0.8µL, with a total volume of 50µL. After the reaction was completed, the magnetic beads were adsorbed by a magnetic rack, and the supernatant was collected.
5. When using transposition complex 2, after one hour of hybridization time was completed, the mixed reaction solution of ligase was added to re-suspend the magnetic beads, and reacted at 20°C for one hour, in which the ligase (T4 DNA ligase, 600U/µL, Enzymatics) was 1µL, the ligation buffer with 10-fold concentration was 20µL, and the volume was adjusted up to 200µL with molecular water. After the reaction was completed, the mixture was washed with a high-salt magnetic bead washing solution and a low-salt magnetic bead washing solution, respectively. Then, 2µLof a USER enzyme (1U/µL, NEB) was added and placed on a vertical mixer to react at 37°C for 0.5 hours. After the reaction was completed, 5µL of 0.44% SDS was added and incubated for 10 minutes at room temperature to denature transposase and release the transposase from DNA, and then washed with the high-salt magnetic bead washing solution and the low-salt magnetic bead washing solution, respectively. Then the magnetic beads were resuspended with a ligase reagent mixture, and placed on a vertical mixer to react at 25°C for 1 hour. The mixed solution of the ligase reagent contained 5µL of ligase (T4 DNA ligase, 600U/µL, Enzymatics), 10L of 3-fold concentration of ligation buffer (polyethylene glycol 8000 (PEG8000)) (30%), Tris-HCl (150mM), ATP (1mM), bovine serum albumin (BSA) (0.15mg/mL), MgCl₂ (magnesium chloride, 30mM), dithiothreitol (DTT, 1.5 mM), and 1.5µL of 16.7µM linker 2 that was formed by annealing sense linker 2-F and antisense linker 2-R), with a total volume of 30µL. After the reaction was completed, the mixture was with the high-salt magnetic bead washing solution and the low-salt magnetic bead washing buffer, respectively. Then the mixed solution of the polymerase reagent and 1µL of primer 2 (100µM) was added to re-suspend the magnetic beads, and reacted at 72°C for 10 minutes. The polymerase (Standard Taq polymerase buffer, 5U/µL, NEB) was 1µL, 10× thermopol buffer (thermopol buffer of 10-fold concentration, NEB company, 200Mm Tris-HCl, 100mM (NH₄)₂SO₄ (ammonium sulfate), 100mM KCl (potassium chloride), 20mM MgSO₄ (magnesium sulfate), and 1% Triton® X-100) was 5µL, 25mM dNTP (Enzymatics) was 0.8µL, with a total volume of 50µL. After the reaction was completed, the magnetic beads were adsorbed by a magnetic rack, and the supernatant was collected.
   The linker 2-F: 5'phos-TCTGCTGAGTCGAGAACGTCTddC-3' (SEQ ID NO: 17);
   The linker 2-R: 5'-CTCGACTCAGCAGddA-3' (SEQ ID NO: 18);
   Primer 2: 5'phos-GAGACGTTCTCGACTCAGCAGA-3' (SEQ ID NO: 19).
6. When the carrier magnetic beads of the scheme 2 and transposition complex 1 were used, hybridization and ligation reactions were omitted. 1.25µL of 0.44% SDS was added into the product of step 2 and the mixture was incubated for 10 minutes at room temperature to denature transposase and release transposase from DNA, and then washed with a high-salt magnetic bead washing solution and a low-salt magnetic bead washing solution, respectively. 2µL of a USER enzyme (1U/µL NEB) was added, the mixture was placed on a vertical mixer to react at 37°C for 0.5 hours, and then washed with the high-salt magnetic bead washing solution and the low-salt magnetic bead washing buffer preheated at 37°C, respectively. Then, the mixed solution of a polymerase reagent was added to re-suspend the magnetic beads, and reacted at 72°C for 10 minutes. The polymerase (Standard Taq polymerase, 5U/µL, NEB) was 1µL, the 10xthermopol buffer (thermopol buffer of 10-fold concentration, NEB, 200mM Tris-HCl, 100mM (NH₄)₂SO₄ (ammonium sulfate), 100mM KCl (potassium chloride), 20mM MgSO₄ (magnesium sulfate), and 1% Triton® X-100) was 5µL, the 25mM dNTP (Enzymatics) was 0.8µL, with a total volume of 50µL. After the reaction was completed, magnetic beads were adsorbed by a magnetic rack, and the supernatant was collected.
7. When the carrier magnetic beads of the scheme 2 and the transposition complex 2 were used, hybridization and ligation reactions were omitted. 1µL of a USER enzyme (1U/µL, NEB) was added to the product of step 2, and placed on a vertical mixer to react at 37°C for 0.5 hours. After the reaction was completed, 1.25µL of 0.44% SDS was added and incubated at room temperature for 10 minutes to denature transposase and release the transposase from DNA, and then washed with high-salt magnetic bead washing solution and low-salt magnetic bead washing solution, respectively. Then, the magnetic beads were resuspended with a ligase reagent mixture, and placed on a vertical mixer to react at 25°C for 1 hour. The mixed solution of the ligase reagent contained 5µL of ligase (T4 DNA ligase, 600U/µL, Enzymatics), 10µL of a ligation buffer with 3-fold concentration (polyethylene glycol 8000 (PEG8000), 30%), Tris-HCl (Tris-hydrochloric acid, 150mM), ATP 1mM, bovine serum albumin (BSA) 0.15mg/mL, MgCl₂ 30mM, dithiothreitol (DTT, 1.5mM), and 1.5µL of 16.7µM linker 2 that was formed by annealing sense linker 2-F and antisense linker 2-R, with a total volume of 30µL. After the reaction was completed, the mixture was washed with the high-salt magnetic bead washing solution and the low-salt magnetic bead washing buffer, respectively. Then, the mixed solution of the polymerase reagent and 1µL of primer 2 (100µM) was added to re-suspend the magnetic beads, and reacted at 72°C for 10 minutes. The polymerase (Standard Taq polymerase, 5U/µL, NEB) was 1µL, the 10xthermopol buffer (thermopol buffer of 10-fold concentration, NEB, 200mM Tris-HCl, 100mM (NH₄)₂SO₄ (ammonium sulfate), 100mM KCl (potassium chloride), 20mM MgSO₄ (magnesium sulfate), and 1% Triton® X-100) was 5µL, 25mM dNTP (Enzymatics) was 0.8µL, with a total volume of 50µL. After the reaction was completed, magnetic beads were adsorbed by magnetic rack, and the supernatant was collected.
8. The DNA molecule polymerase chain amplification primer 1 was repeated for 5-8 cycles using primer 1 and primer 2. The amplification reagent was TD601 PCR kit (Vazyme Biotech Co. Ltd). After amplification, it was purified with XP magnetic beads (Agencourt AMPure XP-Medium, A63882, AGENCOURT). The purification method was performed in accordance with the official standard operation instruction. After purification, the collected product was short fragment molecules having the molecular barcodes that are suitable for sequencing.
   Primer 1: 5'-TGTGAGCCAAGGAGTTG-3' (SEQ ID NO:20);
   Primer 2: 5'phos-GAGACGTTCTCGACTCAGCAGA-3' (SEQ ID NO:21).
8. Sequencing was performed with bgisenq-500, the library obtained in the previous step was subjected to single-strand cyclization reaction. For operation details, please refer to the cyclization step of the BGIseq-500 standard DNA fragment creation process.
9. Through molecular barcode information, the short fragment information obtained by sequencing was restored to the long fragment information of cDNA, to obtain the mRNA expression level.

### Experimental results:

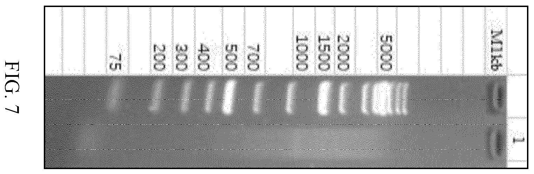

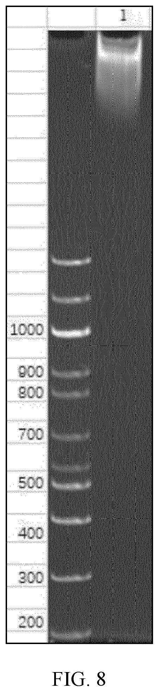

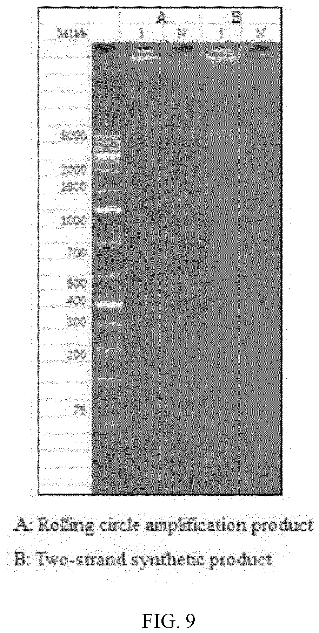

1. The results of the preparation of full-length transcripts: electrophoresed using 1.0% agarose gel at a voltage of 140V for 45 minutes. The results are shown in FIG. 7.
2. The full-length transcript product was subjected to double-strand cyclization, and the cyclized product was electrophoresed with 6% polyacrylamide gel at a voltage of 200V for 30 minutes. The results are shown in FIG. 8.
3. The cyclized product was subjected to rolling circle amplification, and the product was electrophoresed with 5% agarose gel at a voltage of 140V for 45 minutes. The results are shown in FIG. 9.
4. The rolling circle amplification product was subjected to two-strand synthesis, and the synthesized product was electrophoresed with 1.5% agarose gel at a voltage of 140V for 45 minutes. The results are shown in FIG. 9.

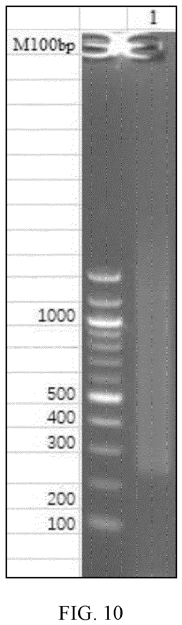

By using the sample of transposon complex 1, 210ng of small fragment having a barcode sequence was finally obtained and electrophoresed for 45 minutes with 1.5% agarose gel at a voltage of 1XTAE 120V, and the results are shown in FIG.10, in which the bands were between 250-3000bp, and the main band was about at 500bp. According to the conversion relationship between DNA quality and molar, 210ng DNA was 636fmol (210/660/500*1000*1000), meeting the requirements of standard BGIseq-500 cyclization step. After cyclization, 19ng (200fmol) of single-stranded ring was obtained, meeting the sequencing requirements.

The above specific examples are applied to illustrate the present disclosure, merely for facilitating the understanding of the present disclosure, instead of limiting the present disclosure. Those skilled in the technical field of the present disclosure, according to the concept of the present disclosure, can make various simple derivations, modifications or substitutions.

## Claims

1. A method for constructing a sequencing library, the method comprising:
cyclizing a linear nucleic acid molecule to form a circular nucleic acid molecule, performing a rolling circle amplification using the circular nucleic acid molecule as a template to obtain a multi-copy long-fragment nucleic acid molecule, and then synthesizing a complementary strand to obtain a double-stranded long-fragment nucleic acid molecule;
mixing and incubating the long-fragment nucleic acid molecule with a transposition complex to form a long-fragment nucleic acid molecule having the transposition complex, wherein the transposition complex comprises a transposon sequence and a transposase; and then mixing and incubating with a molecular barcode sequence on a solid-phase carrier so as to connect the molecular barcode sequence to the transposon sequence of the transposition complex;
releasing the transposase of the transposition complex from the long-fragment nucleic acid molecule, to break the long-fragment nucleic acid molecule into a plurality of short-fragment nucleic acid molecules, wherein each the plurality of short-fragment nucleic acid molecules is connected with the transposon sequence and the molecular barcode sequence, and the plurality of short-fragment nucleic acid molecules derived from the same long-fragment nucleic acid molecule is connected with the same molecular barcode sequence.

2. A method for constructing a sequencing library, the method comprising:
cyclizing a linear nucleic acid molecule to form a circular nucleic acid molecule, performing a rolling circle amplification using the circular nucleic acid molecule as a template to obtain a multi-copy long-fragment nucleic acid molecule, and then synthesizing a complementary strand to obtain a double-stranded long-fragment nucleic acid molecule;
connecting a molecular barcode sequence on a solid-phase carrier having the molecular barcode sequence to a transposon sequence, then mixing and incubating with a transposase and optionally another transposon sequence in such a manner that the transposon sequence and the transposase form a transposition complex to obtain the solid-phase carrier having the molecular barcode sequence and the transposition complex, and then mixing and incubating with the long-fragment nucleic acid molecule to connect the transposition complex with the long-fragment nucleic acid molecule;
releasing the transposase of the transposition complex from the long-fragment nucleic acid molecule, to break the long-fragment nucleic acid molecule into a plurality of short-fragment nucleic acid molecules, wherein each of the plurality of short-fragment nucleic acid molecules is connected with the transposon sequence and the molecular barcode sequence, and the plurality of short-fragment nucleic acid molecules derived from the same long-fragment nucleic acid molecule is connected with the same molecular barcode sequence.

3. The method for constructing a sequencing library according to claim 1 or 2, the method further comprising:
amplifying, through polymerase chain reaction, the short-fragment nucleic acid molecule connected with the transposon sequence and the molecular barcode sequence in such a manner that each molecule of an amplification product comprises the short-fragment nucleic acid molecule, the transposon sequence, and the molecular barcode sequence.

4. The method for constructing a sequencing library according to claim 1 or 2, wherein the linear nucleic acid molecule is a nucleic acid molecule in a Formalin-Fixed and Paraffin-Embedded sample, or a cDNA sequence after reverse transcription of a full-length mRNA, or a full-length DNA sequence after reverse transcription of 18S rRNA or 16S rRNA, or a genomic DNA fragment sequence, or a full-length sequence of a mitochondrial or small genome sequence, or an amplicon sequence of a target region of a genomic DNA.

5. The method for constructing a sequencing library according to claim 1 or 2, wherein the linear nucleic acid molecule is cyclized to form the circular nucleic acid molecule by connecting a linker sequence at two terminals and forming complementary sticky terminals at the two terminals, and then the multi-copy long-fragment nucleic acid molecule is obtained through the rolling circle amplification using the circular nucleic acid molecule as the template and a sequence complementary to the linker sequence as a primer.

6. The method for constructing a sequencing library according to claim 5, wherein the linker sequence comprises a U base site, and the complementary sticky terminals are formed by USER enzyme digestion; or
the linker sequence comprises an enzyme digestion site, and the complementary sticky terminals are formed by means of enzyme digestion.

7. The method for constructing a sequencing library according to claim 1 or 2, wherein the transposition complex comprises a pair of transposon sequences that are identical to or different from each other.

8. The method for constructing a sequencing library according to claim 7, wherein the transposition complex comprises the pair of transposon sequences that are different from each other, each transposon sequence comprises a sense strand and an antisense strand,
wherein in one transposon sequence of the pair of transposon sequences, the sense strand is connectable with the molecular barcode sequence, and the antisense strand has a U base site, which is removable by USER enzyme digestion to facilitate a subsequent polymerase chain reaction amplification.

9. The method for constructing a sequencing library according to claim 7, wherein the transposition complex comprises the pair of transposon sequences that are identical to each other, each transposon sequence comprises a sense strand and an antisense strand, and the sense strand of each transposon sequence is connectable with the molecular barcode sequence, and the antisense strand of each transposon sequence comprises a U base site, which is removable by USER enzyme digestion.

10. The method for constructing a sequencing library according to claim 9, wherein after the transposase of the transposition complex is released from the long-fragment nucleic acid molecule to break the long-fragment nucleic acid molecule into the plurality of short-fragment nucleic acid molecules, a second linker sequence is connected at a gap where the transposon sequence is connected to the short-fragment nucleic acid molecules, and then polymerase chain reaction amplification is performed.

11. The method for constructing a sequencing library according to claim 1 or 2, wherein the solid phase carrier having the molecular barcode sequence comprises more than two molecular barcode sequences.

12. The method for constructing a sequencing library according to claim 1 or 2, wherein the molecular barcode sequence is added to the solid phase carrier by connecting with the linker sequence on the solid phase carrier.

13. The method for constructing a sequencing library according to claim 1 or 2, wherein the solid phase carrier having the molecular barcode sequence comprises more than two molecular barcode sequences, and the more than two molecular barcode sequences are sequentially connected and added to the solid phase carrier to form a combined molecular barcode comprising the more than two molecular barcode sequences.

14. The method for constructing a sequencing library according to claim 1, wherein before mixing and incubating the long-fragment nucleic acid molecule having the transposition complex with the solid phase carrier having the molecular barcode sequence, a transposition complex-capturing sequence is added to the solid phase carrier having the molecular barcode sequence to complementarily connect to the molecular barcode sequence, the transposition complex-capturing sequence is then mixed and incubated with the long-fragment nucleic acid molecule having the transposition complex in such a manner that the transposition complex-capturing sequence is complementary to the molecular barcode sequence and the transposon sequence of the transposition complex to form a bridge therebetween, and the molecular barcode sequence is connected to the transposon sequence of the transposition complex under effect of a ligase.

15. The method for constructing a sequencing library according to claim 1, wherein when the long-fragment nucleic acid molecule having the transposition complex is mixed and incubated with the solid phase carrier having the molecular barcode sequence, each solid phase carrier forms a virtual division in such a manner that one solid phase carrier captures one long-fragment nucleic acid molecule having the transposition complex and connects the molecular barcode sequence with the transposon sequence of the transposition complex.

16. The method for constructing a sequencing library according to claim 2, wherein when the solid-phase carrier having the molecular barcode sequence and the transposition complex is mixed and incubated with the long-fragment nucleic acid molecule, each solid-phase carrier forms a virtual division in such a manner that one solid-phase carrier captures one long-fragment nucleic acid molecule.

17. A sequencing library prepared by the method according to any one of claims 1 to 16.

18. A sequencing method, comprising sequencing the sequencing library prepared by any one of claims 1 to 16.

19. The sequencing method according to claim 18, wherein said sequencing is selected from full-length transcript assembly sequencing, full-length sequencing of 18S rRNA or 16S rRNA, full-length sequencing of mitochondria, or long-fragment amplicon sequencing.


SEQUENCE LISTING

<110>  BGI SHENZHEN

<120>  METHOD FOR CONSTRUCTING SEQUENCING LIBRARY, OBTAINED SEQUENCING
LIBRARY AND SEQUENCING METHOD

<130>  18P26890

<160>  21

<170>  PatentIn version 3.3

<210>  1
<211>  27
<212>  DNA
<213>  Artificial sequence
<220>
<223>  Synthetic sequence

<400>  1
aaaaaaaaaa tgtgagccaa ggagttg                                          27


<210>  2
<211>  23
<212>  DNA
<213>  Artificial sequence
<220>
<223>  Synthetic sequence

<400>  2
ccagagcaac tccttggctc aca                                              23


<210>  3
<211>  37
<212>  DNA
<213>  Artificial sequence
<220>
<223>  Synthetic sequence


<220>
<221>  misc_feature
<222>  (22)..(31)
<223>  n is a, c, g, or t

<400>  3
ctctggcgac ggccacgaag cnnnnnnnnn ntctgcg                               37


<210>  4
<211>  25
<212>  DNA
<213>  Artificial sequence
<220>
<223>  Synthetic sequence


<220>
<221>  misc_feature
<222>  (1)..(10)

<223>  n is a, c, g, or t

<400>  4
nnnnnnnnnn gcttcgtggc cgtcg                                          25


<210>  5
<211>  25
<212>  DNA
<213>  Artificial sequence
<220>
<223>  Synthetic sequence


<220>
<221>  misc_feature
<222>  (1)..(10)
<223>  n is a, c, g, or t

<400>  5
nnnnnnnnnn tagcatggac tatgg                                          25


<210>  6
<211>  26
<212>  DNA
<213>  Artificial sequence
<220>
<223>  Synthetic sequence


<220>
<221>  misc_feature
<222>  (11)..(20)
<223>  n is a, c, g, or t

<400>  6
gtccatgcta nnnnnnnnnn cgcaga                                         26


<210>  7
<211>  43
<212>  DNA
<213>  Artificial sequence
<220>
<223>  Synthetic sequence

<400>  7
atcgtacctg ataccgctag gaaccactag tacagcagtc acg                      43


<210>  8
<211>  58
<212>  DNA
<213>  Artificial sequence
<220>
<223>  Synthetic sequence

<400>  8
cgatccttgg tgatcatgtc gtcagtgctt gtcttcctaa gatgtgtata agagacag      58


<210>  9

<211>  19
<212>  DNA
<213>  Artificial sequence
<220>
<223>  Synthetic sequence

<400>  9
ctgtctctta tacacatct                                                    19


<210>  10
<211>  41
<212>  DNA
<213>  Artificial sequence
<220>
<223>  Synthetic sequence

<400>  10
gagacgttct cgactcagca gaagatgtgt ataagagaca g                           41


<210>  11
<211>  19
<212>  DNA
<213>  Artificial sequence
<220>
<223>  Synthetic sequence

<400>  11
ctgtctctta tacacatct                                                    19


<210>  12
<211>  58
<212>  DNA
<213>  Artificial sequence
<220>
<223>  Synthetic sequence


<220>
<221>  misc_feature
<222>  (27)..(36)
<223>  n is a, c, g, or t

<400>  12
aagcttcgta gccatgtcgt tctgcgnnnn nnnnnntttt tttttttttt tttttttv        58


<210>  13
<211>  27
<212>  DNA
<213>  Artificial sequence
<220>
<223>  Synthetic sequence

<400>  13
aagcttcgta gccatgtcgt tctgggg                                           27


<210>  14
<211>  24
<212>  DNA

<213>  Artificial sequence
<220>
<223>  Synthetic sequence

<400>  14
aagcttcgta gccatgtcgt tctg                                              24


<210>  15
<211>  16
<212>  DNA
<213>  Artificial sequence
<220>
<223>  Synthetic sequence

<400>  15
tttttttttt tttttt                                                       16


<210>  16
<211>  18
<212>  DNA
<213>  Artificial sequence
<220>
<223>  Synthetic sequence

<400>  16
cgtagccatg tcgttctg                                                     18


<210>  17
<211>  22
<212>  DNA
<213>  Artificial sequence
<220>
<223>  Synthetic sequence

<400>  17
tctgctgagt cgagaacgtc tc                                                22


<210>  18
<211>  14
<212>  DNA
<213>  Artificial sequence
<220>
<223>  Synthetic sequence

<400>  18
ctcgactcag caga                                                         14


<210>  19
<211>  22
<212>  DNA
<213>  Artificial sequence
<220>
<223>  Synthetic sequence

<400>  19
gagacgttct cgactcagca ga                                                22

<210>  20
<211>  17
<212>  DNA
<213>  Artificial sequence
<220>
<223>  Synthetic sequence

<400>  20
tgtgagccaa ggagttg                                                    17


<210>  21
<211>  22
<212>  DNA
<213>  Artificial sequence
<220>
<223>  Synthetic sequence

<400>  21
gagacgttct cgactcagca ga                                              22